# EUROPEAN PATENT APPLICATION

(11) **EP 1 442 754 A1**
(43) Date of publication of application: **04.08.2004**
(21) Application number: 03079186.7
(22) Date of filing: 24.12.2003
(51) Int. Cl.: A61L 9/12, B60H 3/00

(54) **Device for diffusing vapours into the environment**

(30) Priority: 31.01.2003 IT RE20030011
(71) Applicant: RE.LE.VI. S.p.a., 46040 Rodigo (Mantova) (IT)
(72) Inventor: Pagani, Fabio, c/o RE.LE.VI.-S.P.A., 46040 Rodigo (Mantova) (IT)
(74) Representative: Corradini, Corrado

(57) **Abstract**

The device comprises: a bottle (10) for the active liquid, having a mouth (11) with an exit hole (12); a diffuser vessel (20) embracing and surrounding the lower part of the bottle (10) to define together therewith a chamber (24) within which the mouth of the bottle (10) is enclosed and communicates with the external environment for vapour diffusion; absorbent means (25, 26) positioned in the interior of said chamber (24) defined by the diffuser vessel, to absorb the active liquid and to release it as vapour. The bottle (10) and the diffuser vessel (20) are disposed, when in use, in an inverted state such as to locate the mouth (11) in a lower end position. The diffuser vessel (20) possesses, relative to the bottle (10), a first stable axial position in which the mouth of the bottle (10) is further from the base wall (21) of the vessel (20), and a second stable axial position in which the mouth of the bottle (10) is closer to said base wall (21), and also comprises a valve (30) applied to the mouth of the bottle (10) to close the exit hole (12) when the diffuser vessel (20) is in said first axial position, and to open said hole (12) when the diffuser vessel (20) is brought into said second axial position. By virtue of the invention the exit hole (12) can be selectively closed even after the removal of any seal provided to maintain the bottle (10) sealed before its purchase by the user.

## Description

This invention relates to a device for diffusing vapours into the environment, in particular for deodorizing and perfuming domestic or work rooms.

Vapour diffusers, in particular for room deodorants, have been known for a long time, they comprising a bottle containing an active liquid able to evaporate into the environment to diffuse vapours, and having a mouth which is provided with an exit hole for the active liquid and is enclosed within a diffuser vessel applied to the bottle such as to surround the mouth and be secured to it in axially variable positions; inside the diffuser vessel there are also placed absorbent means able to absorb the active liquid and to release it as vapour, these being the disposed, in relation to the bottle mouth, in such a manner as to absorb all the liquid leaving the bottle. The chamber of the diffuser vessel communicates with the external environment via at least one aperture for diffusion of the vapour.

In use, after removing a seal positioned by the producer to hermetically seal the exit hole, the user positions the bottle in an inverted state, together with the diffuser vessel, such that the mouth lies in a lower position, and in this condition the active liquid leaves in a gauged manner through the exit hole and impregnates the absorbent means, which release vapours which finally diffuse into the external environment.

An object of this invention is to offer a diffuser device provided with means which enable the user, after the seal has been removed, to close and to reopen the bottle exit hole at will, so enabling vapour diffusion to be interrupted for any period, this being achieved by simple, reliable and constructionally low-cost means.

This and other objects are attained by the device of the invention as characterised in the claims.

The invention is described in detail hereinafter with the aid of the accompanying figures, which illustrate one embodiment thereof by way of non-exclusive example.

Figure 1 is an exploded perspective view of a first embodiment of the device of the invention.

Figure 2A is a section through the device of Figure 1 on a vertical axial plane, the diffuser vessel 20 being in its first axial position.

Figure 2B is a section as in Figure 1, the diffuser vessel 20 being in its second axial position.

Figure 3A is an enlarged detail of Figure 2A.

Figure 3B is an enlarged detail of Figure 2B.

Figure 4A is a section on a vertical axial plane through a second embodiment of the device, in which the diffuser vessel 20 is in the first axial position.

Figure 4B is a section similar to Figure 4A, in which the diffuser vessel 20 is in the second axial position.

Figure 5A is an enlarged detail of Figure 4A.

Figure 5B is an enlarged detail of Figure 4B.

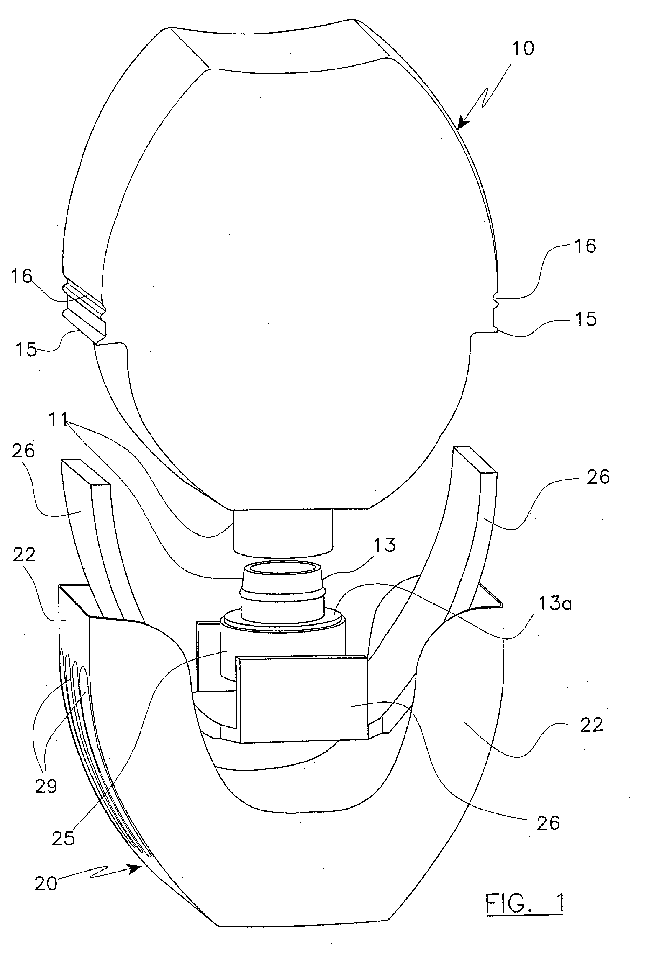

The device illustrated in Figures 1, 2A, 2B, 3A and 3B comprises a bottle 10 for containing an active liquid able to evaporate into the environment to diffuse vapours (for example perfuming or disinfectant vapours) and possessing a mouth 11 provided with the exit hole 12 for the active liquid.

With the bottle 10 there is associated a diffuser vessel 20 which in the embodiment illustrated in the figures, has a horizontal flat wall 21 acting as a resting base for the entire diffuser, and a side wall 22 which embraces and surrounds the lower part of the bottle, to define together therewith a chamber 24 within which the mouth 11 of the bottle 10 is enclosed.

In use, the bottle 10 and the diffuser vessel are disposed in an inverted state to bring the mouth 11 into a lower end position. In this inverted state, the diffuser vessel 20 acts as a support for the bottle 10.

The ensuing description assumes that the dispenser is disposed in its condition for use, with the bottle containing the active substance in the inverted state.

In detail, in the embodiment illustrated in the figures, the mouth 11 is of cylindrical bush shape and is closed by a press-in plug 13 having a lower horizontal wall 13a which closes the lower end of the bush 11, and in which the exit hole 12 is provided.

Absorbent means are positioned in the interior of the chamber defined by the diffuser vessel, to absorb the active liquid and to release it as vapour, they being disposed, in relation to the bottle mouth, in such a manner as to absorb the liquid leaving the mouth. In particular, these absorbent means comprise an elastically deformable spongy absorbent body 25, of small cylinder shape, which covers the lower wall 13a and closes the mouth 11 of the bottle 10, and an absorbent layer 26 placed along the inner surface of the diffuser vessel 20, both on its base wall 21 and on its side wall 22, the absorbent body 25 being in contact with the absorbent layer 26 either constantly (as shown in the figures) or at least when in its second axial position described hereinafter.

The side wall 22 of the diffuser vessel 20 is secured to the side walls of the bottle 10 in axially variable positions.

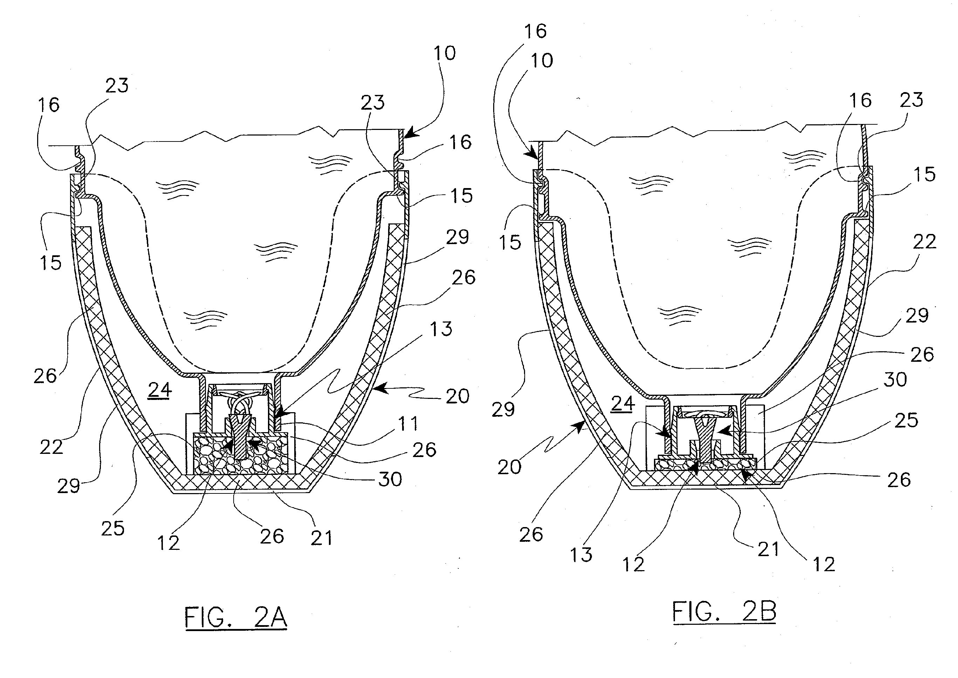

In particular, according to the invention, the diffuser vessel 20 possesses, relative to the bottle 10, a first stable axial position (shown in Figure 2A) in which the bottle mouth 11 is further from the base wall 21 of the vessel 20, and a second stable axial position (shown in Figure 2B) in which the bottle mouth 11 is closer to the base wall 21.

In detail, the side wall 22 of the diffuser vessel 20 possesses, close to its upper edge, two inwardly facing projections 23 positioned on two opposing sides, to engage a first pair of outwardly facing relative projections 15 positioned on two corresponding side walls of the bottle 10 or, selectively, a pair of concave seats 16 provided on the same side walls of the bottle 10 but positioned slightly higher than the projections 15.

The projections 23 are arranged to elastically snap-engage in the seats 16, and when this occurs the bottle 10 is maintained in said second axial position, in which the mouth 11 of the bottle 10 is so close to the base wall 21 as to compress the absorbent body 25 to a maximum extent against the lower and central region of the layer 26 (see Figure 2B), so reducing its thickness.

The projections 23 are also arranged to engage with and abut against the upper surface of the projections 15 when the bottle is in said first axial position, when in this axial position (see Figure 2A) the bottle being constrained not to upwardly withdraw from the diffuser vessel 20; said bottle 10 cannot further approach the base wall 21 as the absorbent body 25 abuts against the absorbent layer 26.

The chamber 24 communicates with the external environment via at least one aperture, in particular via several apertures 29 provided in the side walls of the diffuser vessel 20, which enable the vapour formed in the interior of the chamber 24 to diffuse into the external environment.

According to the invention, the diffuser also comprises a valve 30 applied to the bottle mouth 11 to close the exit hole 12 when the diffuser vessel 20 is in said first axial position, and to open said hole 12 when the diffuser vessel 20 is brought into said second axial position.

In the embodiment shown in the figures, the valve 30 possesses a valving element 31, particularly of frusto-conical shape, which normally closes the exit hole 12, and a rod 32 acting on the valving element 31 and passing through the exit hole 12, said rod being displaced as a result of contact either with the absorbent means 25, 26 or directly with the base wall 21 of the diffuser vessel 20, when this latter is positioned in said second axial position, so that the valving element 31 moves away from the exit hole 12.

Said rod 32 is fixed axially to the lower end of the valving element 31, and projects downwards from the exit hole 12, being incorporated into said absorbent body 25, which is fixed to the wall 13a.

The valve possesses spring means 33 acting on the valving element 31 to normally urge it against a suitable seat 12a provided in the exit hole 12, to close the hole. Said spring means 33 are formed from a number of spiral filaments, obtained during the moulding of the valve, which are joined at their upper end to a connection ring 34 fixed to the upper mouth of the plug 13, and at their other end to the valving element 31.

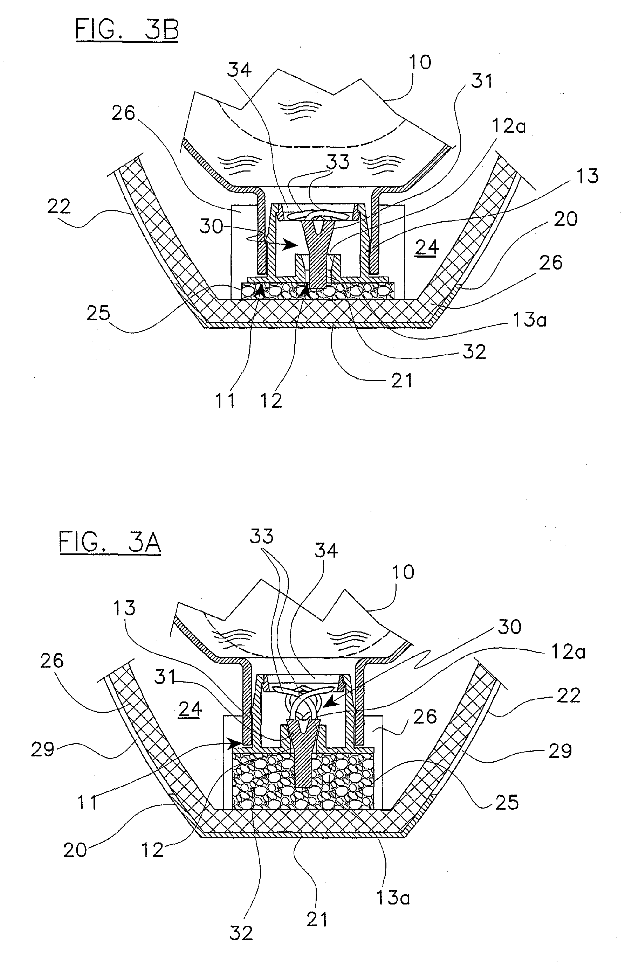

When the mouth 11 is at its maximum distance from the base wall 21 (Figure 3A - first axial position), the rod 32 is not subjected to axial thrust and consequently the valving element 31, by virtue of the thrust of the filaments 33 (in addition to its own weight), closes the seat 12a of the hole 12. This happens with the bottle 10 both in its upright state, and in its inverted state (as shown in the figures); when in this second state the weight of the liquid present in the bottle 10 urges the valving element 31 downwards, making the closure condition more stable.

In contrast, when the mouth 11 is brought to its minimum distance from the base wall 21 (Figure 3B - second axial position), the absorbent body 25 is compressed against the layer 26 lying on the wall 21 so that its thickness is considerably decreased; the lower end of the rod 32 therefore abuts against the layer 26 (the body 25 being interposed in compressed form) and is consequently raised relative to the mouth 11; the valving element 31 is therefore raised from the seat 12a, so opening the hole 12, the liquid present in the bottle then descending along the hole 12 to firstly impregnate the body 25 and then the layer 26, from which it escapes in vapour form, to finally emerge into the external environment.

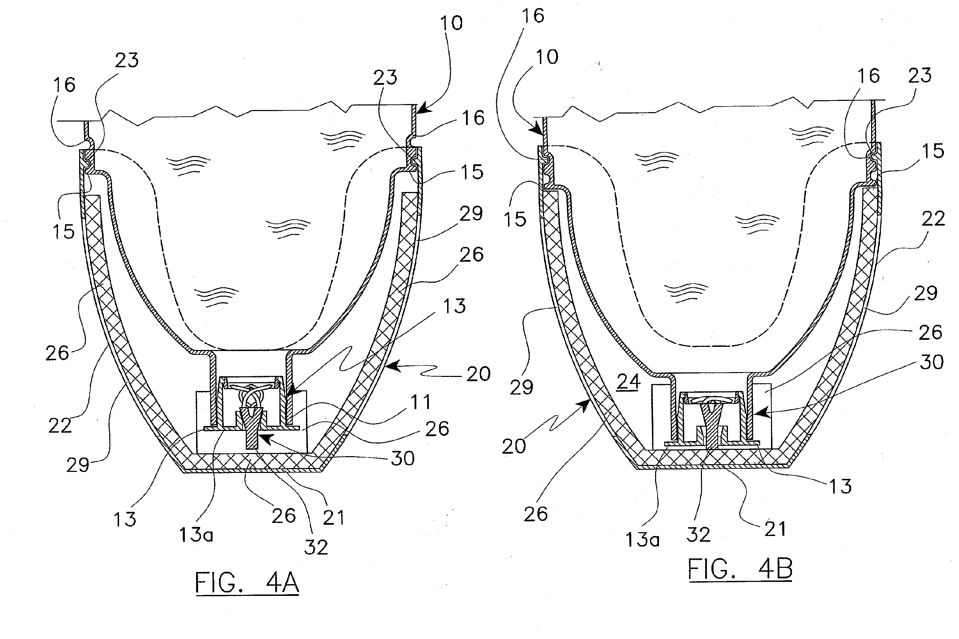

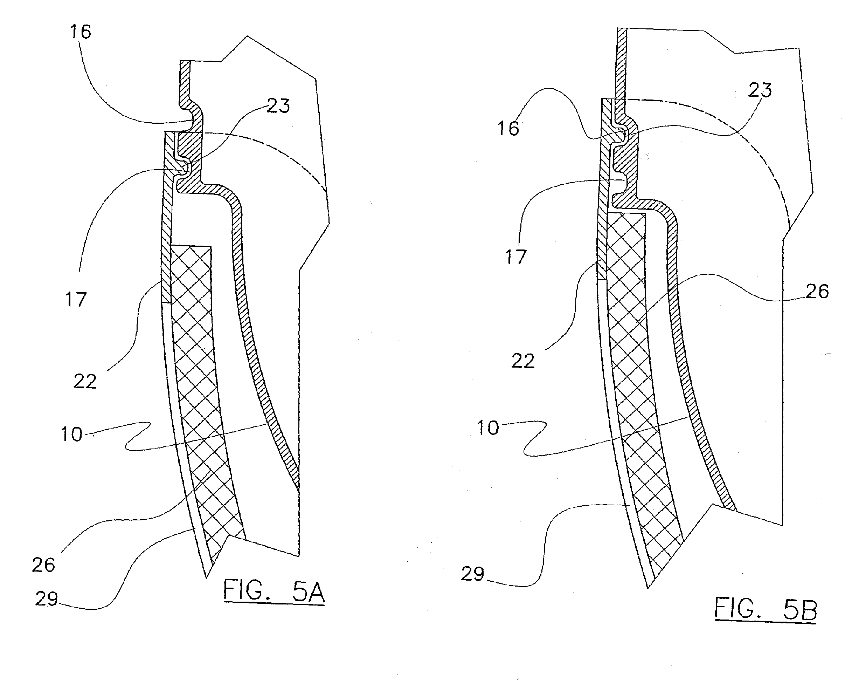

Figures 4A, 4B, 5A and 5B show a second embodiment which differs from the aforedescribed first embodiment in that no element (in particular the absorbent body 25) is applied to the lower wall 13a of the plug 13, hence the rod 32 of the valving element 31 projects axially and freely downwards.

When the mouth 11 is at its maximum distance from the base wall 21 (Figure 4A - first axial position), the rod 32 is spaced from the absorbent layer 26 positioned on the wall 21 and is not subjected to axial thrusts, consequently the valving element 31 closes the seat 12a of the hole 12. This happens both if the bottle 10 is in its upright state and in its inverted state.

However when the mouth 11 is brought to its minimum distance from the base wall 21 (Figure 4B - second axial position), the lower end of the rod 32 abuts against the layer 26 on the wall 21, the valving element 31 being consequently lifted from its seat 12a, to hence leave the hole 12 open.

To maintain the bottle 10 in said first position, in contrast to the solution shown in Figure 2A, a pair of second concave seats 17 are provided, positioned on the same lateral sides of the bottle 10 as the first seats 16 but slightly below these (see Figures 5A and 5B). Said first position is defined when the projections 23 are made to elastically snap-engage in the second seats 17; instead, when they engage in the first seats 16, said second position is defined.

A solution such as that shown in Figures 5A and 5B can also be provided for the aforedescribed first embodiment.

By virtue of the invention the exit hole 12 can be selectively closed even after the removal of any seal preferably provided to maintain the bottle 10 sealed before its purchase by the user, so as to block delivery of the liquid substance from the bottle 10 if the dispenser is in its inverted state, or of the vapour alone if the dispenser is in its upright state; this is achieved by simply displacing the diffuser vessel 20 in such a manner as to withdraw the mouth 11 from the base wall 21 (by moving it into its first axial position).

This aspect is advantageous in preserving the active liquid during those periods in which the dispenser is not used.

Vice versa, to activate vessel diffusion the mouth 11 has merely to be moved closer to the diffuser vessel 20, to hence open the exit hole 12, and the dispenser put into its inverted state.

Another advantage is that the liquid leaves the bottle through the absorbent body 25, and even if laid on its side, delivery takes place in the same manner as if it were in a vertical position.

Numerous modifications of a practical and applicational nature can be made to the device of the invention, but without leaving the scope of the inventive idea as claimed below.

## Claims

1. A device for diffusing vapours into the environment, comprising:
- a bottle (10) for containing an active liquid able to diffuse vapours into the environment, and having a mouth (11) provided with an exit hole (12) for the active liquid,
- a diffuser vessel (20) embracing and surrounding the lower part of the bottle (10) to define together therewith a chamber (24) within which the mouth of the bottle (10) is enclosed and communicates with the external environment for vapour diffusion,
- absorbent means (25, 26) positioned in the interior of said chamber (24) defined by the diffuser vessel, to absorb the active liquid and to release it as vapour, said absorbent means being disposed, in relation to the mouth of the bottle (10), in such a manner as to absorb the liquid leaving the mouth,
- said bottle (10) and said diffuser vessel (20) being disposed, when in use, in an inverted state such as to locate the mouth (11) in a lower end position,
**characterised in that** the diffuser vessel (20) possesses, relative to the bottle (10), a first stable axial position in which the mouth of the bottle (10) is further from the base wall (21) of the vessel (20), and a second stable axial position in which the mouth of the bottle (10) is closer to said base wall (21), and also comprises a valve (30) applied to the mouth of the bottle (10) to close the exit hole (12) when the diffuser vessel (20) is in said first axial position, and to open said hole (12) when the diffuser vessel (20) is brought into said second axial position.

2. A diffuser device as claimed in claim 1, **characterised in that** said valve (30) possesses a valving element (31) which normally closes the exit hole (12), and a rod (32) acting on the valving element (31) and passing through the exit hole (12), said rod (32) being displaced as a result of contact either with the absorbent means (25, 26) or directly with the base wall (21) of the diffuser vessel (20), when this latter is positioned in said second axial position, so that the valving element (31) moves away from the exit hole (12).

3. A diffuser device as claimed in claim 2, **characterised in that** said valve (30) possesses spring means (33) acting on the valving element (31) to normally urge said valving element (31) against a suitable seat (12a) provided in the exit hole (12) to close the hole (12).

4. A diffuser device as claimed in claim 1, **characterised in that** said absorbent means comprise an absorbent body which covers the mouth of the bottle (10).

5. A diffuser device as claimed in claim 4, **characterised in that** said absorbent means (25, 26) comprise, in addition to said absorbent body (25), an absorbent layer (26) positioned along the inner surface of the diffuser vessel (20), said absorbent body (25) being in contact with the absorbent layer at least in said second axial position.

6. A diffuser device as claimed in claims 2 and 5, **characterised in that** said rod (32) is fixed axially to the valving element (31), it projecting out of the exit hole (12).

7. A diffuser device as claimed in claim 6, **characterised in that** said rod (32) is incorporated into said absorbent body (25).
